# EUROPÄISCHE PATENTANMELDUNG

(11) **EP 0 769 559 A2**
(43) Veröffentlichungstag der Anmeldung: **23.04.1997**
(21) Anmeldenummer: 96115219.6
(22) Anmeldetag: 23.09.1996
(51) Int. Cl.: C12Q 1/56, G01N 33/86

(54) **Verfahren zur Quantifizierung von aktiviertem Gerinnungsfaktor VII (FVIIa)**

(30) Priorität: 18.10.1995 DE 19538716
(71) Anmelder: BEHRINGWERKE AG, 35001 Marburg (DE)
(72) Erfinder: Römisch, Jürgen, Dr., 35041 Marburg (DE); Stöhr, Hans-Arnold, 35083 Wetter (DE)

(57) **Zusammenfassung**

Die Erfindung betrifft ein Verfahren zur Quantifizierung von Gerinnungsfaktor VIIa (F VIIa) in FVIIa- bzw. F VII/FVIIa-haltigen Lösungen mittels selektiver Bindung an immobilisiertes lösliches Thromboplastin.

## Beschreibung

Die Erfindung betrifft ein Verfahren zur Quantifizierung von Gerinnungsfaktor VIIa (F VIIa) in FVIIa- bzw. F VII/FVIIa-haltigen Lösungen mittels selektiver Bindung an immobilisiertes lösliches Thromboplastin.

Gerinnungsfaktor VII (FVII) stellt zusammen mit dem Thromboplastin (tissue factor, TF) den Initiatorkomplex des exogenen Gerinnungsweges dar. Der physiologisch aktive Anteil des TF-FVII/TF-FVIIa Gemisches, nämlich TF-FVIIa, aktiviert in Gegenwart von (bevorzugt negativ geladenen) Lipiden und Kalzium sehr effektiv den Faktor X (FX zu FXa). FXa wiederum katalysiert (zusammen mit FVa, Lipiden und Kalzium) die Generierung von Thrombin. Die anschließende Bildung von Fibrin stellt (u.a.) einen Wundverschluß sicher. Untersuchungsergebnisse der letzten Dekaden legen die Vermutung nahe, daß erhöhte FVIIa-Spiegel zu prothrombotischen Ereignissen beitragen bzw. sogar deren Ursache sein könnten. Entsprechend ist die (frühzeitige) Detektion und Quantifizierung von Spuren FVIIa in Körperflüssigkeiten, vor allem Plasma, von Interesse.

Mehrere Methoden zur FVIIa-Bestimmung wurden beschrieben: Bei der ersten Methode wird genutzt, daß Spuren FVIIa die Gerinnung in entsprechenden in vitro Testsystemen (z.B. Clotting-Test in FVII-Mangelplasma) effektiver beschleunigen als FVII. Dagegen ist der Unterschied in einem Testsystem, das auf der Aktivierung von FX und dem Nachweis von FXa im Reinsystem (in Gegenwart von komplettem TF/Lipid und chromogenem FXa-Substrat) beruht, zwischen FVII und FVIIa weitaus geringer- dieser Test gibt entsprechend die Gesamtheit an FVII/FVIIa an. Der Quotient der durch diese Tests ermittelten FVII-Gehalte gibt einen Anhaltspunkt über die Anwesenheit von aktiviertem FVIIa (Seligsohn et al. Blood 1978; 52: 978-988). Beide Tests können durch anwesende andere aktivierte Faktoren (z.B. FIIa, FXa, FIXa) gestört bzw. die Ergebnisse verfälscht werden.
Bei einem anderen beschriebenen Verfahren wird ausgenutzt, daß löslicher TF (sTF, extrazelluläre Domäne) über sTF-FVIIa die Fibrinbildung (über FXa, FIIa) induziert, jedoch inert gegenüber Autoaktivierungsprozessen von FVII (gebunden an TF) und Aktivierung durch FXa ist (Morrissey; Thromb. Haemostas. 1995; 74:185-188). Der Einfluß anderer aktivierter Faktoren (s.o.) ist jedoch nicht geklärt.

Die o.g. Tests sind für die Detektion von Spuren FVIIa in Plasmaproben konzipiert, eignen sich nach bisheriger Erfahrung aber nur bedingt zu dessen Detektion in Faktorenkonzentraten. Diese Präparate, wie Prothrombinkomplex-Konzentrate oder FVII-Konzentrate, werden z.B. zur Substitution von Patienten mit angeborenen oder erworbenen Mängeln an Gerinnungsfaktoren verwendet. Spuren aktivierter Faktoren, darunter FVIIa, können im Patienten zu thromboembolischen Komplikationen und Tod führen. Die Detektion und Eliminierung/Blockierung von FVIIa ist daher besonders wichtig. Da in den Präparaten die Gerinnungsfaktoren hoch konzentriert vorliegen, ist die Detektion selektiv von FVIIa erschwert:

**a.)** Spuren anderer aktivierter Faktoren können die Testergebnisse verfälschen (s.o); **b.)** die o.g. Systeme wurden für die Testung von Plasmaproben konzipiert; das als Probe verwendete Konzentrat ändert aufgrund der hohen Konzentrationen an verschiedenen Faktoren die Verhältnisse gegenüber Plasma (im Plasma-Testsystem). Die Tauglichkeit für derartige Quantifizierungen ist bisher nicht belegt und auch sehr wahrscheinlich nicht gegeben.

Die von Godal et al. (Thromb. Res. 1974; 5:773-775) beschriebene Methode nutzt die Tatsache, daß FVIIa in der Kälte durch Antithrombin (AT) III/Heparin inhibiert wird. Durch koagulometrische Messung (in FVII-Mangelplasma) von Ausgangsmaterial (ohne ATIII/Heparin) und inhibierter Lösung wird eine Abschätzung des Gehaltes an FVIIa möglich. Allerdings kann auch diese Methode durch die Anwesenheit anderer aktivierter Faktoren gestört werden. Zudem vollzieht sich die quantitative Inhibition von FVIIa langsam (üblicherweise wird die Probe mit ATIII/Hep. über Nacht bei 4°C inkubiert) und birgt damit das Risiko zusätzlicher Aktivierungen oder Denaturierungen während der Standzeit.

Der vorliegenden Erfindung liegt daher die Aufgabe zugrunde, ein Verfahren zur spezifischen Quantifizierung von FVIIa bereitzustellen, das damit auch zum Nachweis von FVIIa in Plasmakonzentraten geeignet ist.

Die Aufgabe wurde wie folgt gelöst: Aus einer FVIIa- oder FVII/FVIIa-haltigen Lösung wird FVIIa (und FVII) an einen immobilisierten sTF gebunden und nicht gebundene Moleküle durch Waschen der Festphase entfernt. FVIIa wird danach **a.)** direkt durch Zusatz eines geeigneten Substrates quantifiziert oder indirekt durch Zugabe eines physiologischen Substrates (Faktor IX oder X) und dessen anschließende Bestimmung oder **b.)** durch Elution des an den immobilisierten sTF gebundenen FVII/FVIIa und anschließende Bestimmung des FVIIa.
Der Probe kann ggfs. vor Kontakt mit dem immobilisierten sTF eine geeignete Menge AT III/Heparin zugesetzt werden, um die weitere Bildung von FVIIa aus FVII zu verhindern (s.u.). Nach Inkubation kann das Heparin, z.B. mit Protamin, neutralisiert und wie oben beschrieben der FVIIa nachgewiesen werden.

Wir haben gefunden, daß die Verwendung von immobilisiertem sTF die spezifische Quantifizierung von FVIIa möglich macht; mit komplettem TF (lipidhaltig) gelingt dies nicht - auch bei Immobilisierung mittels Bindung an eine Festphase, da Autoaktivierungsreaktionen zu TF-FVIIa und die Aktivierung durch FXa keine verläßliche selektive Messung zuläßt.

Die Kopplung (kovalent oder nicht-kovalent) von sTF an eine geeignete Festphase (Mikrotiterplatte, Röhrchen oder Gelmatrices/Sepharose etc.) kann mittels allgemein bekannter Methoden erfolgen. In einer bevorzugten Ausführung wird sTF mittels eines an die Festphase fixierten Antikörpers (PAK;MAK; Fab etc.) gegen sTF gebunden. Eine weitere bevorzugte Ausführung sieht die Bindung eines Fc-sTF-Fusionsproteins mittels eines an die Festphase gebundenen anti-Fc oder anderen geeigneten Antikörpers bzw. an der Festphase immobilisiertes Protein A oder G vor. Zweckmäßigerweise wird daher Fc-sTF gentechnisch hergestellt, wie in der Europäischen Patentanmeldung EP-A-0 464 533 beschrieben.Diese bevorzugten Ausführungen bieten den Vorteil, daß der sTF in einer definierten und für FVII/FVIIa gut zugänglichen Form präsentiert wird und die anschließende Substratspaltung adäquat erfolgt. Diese Assoziation kann durch geeignete Verfahren in eine kovalente Bindung überführt werden. Dies ist besonders dann von Bedeutung, wenn Fc-sTF immobilisiert wird und Fc-Fragment-haltige oder Immunglobulin-haltige Proben getestet werden.

Faktor VIIa-haltige Proben, wie Körperflüssigkeiten, besonders Plasma, oder Faktorenkonzentrate, wie PPSB- oder FVII-Präparate, werden mit dem o.g. immobilisierten sTF in Gegenwart von Kalzium in Kontakt gebracht, inkubiert, die Probenlösung entfernt und die Festphase (ggfs. mehrmals) gewaschen. Dieses Vorgehen entfernt Bestandteile der Probe (z.B. aktivierte Faktoren etc.), die potentiell die im folgenden beschriebenen Reaktionen stören, verhindern bzw. die Ergebnisse verfälschen könnten.
FVIIa kann durch Zugabe eines geeigneten Substrates quantifiziert werden. Bei dem beschriebenen Verfahren fungiert sTF nicht nur als sehr selektive Bindungsstelle für FVII und FVIIa (was ja auch ein entsprechender anti-FVII Antikörper könnte), sondern er potenziert darüber hinaus (Kofaktor-ähnlich) in Gegenwart von Kalzium auch die amidolytische Aktivität von FVIIa. Als Substrate eignen sich üblicherweise verwendete Peptidsubstrate mit chromogenen oder fluorogenen Gruppen etc., deren Spaltung photometrisch/fluorimetrisch gemessen werden kann. Eine höhere Sensitivität des Testsystemes- und eine physiologisch relevantere Aussage- erhält man, wenn natürliche Substrate, wie FIX und/oder FX zugesetzt werden. Die Anwesenheit von Kalzium und gerinnungsaktiven Lipiden beschleunigt die Aktivierung von FIX und FX erheblich und sie werden daher in einem bevorzugten Verfahren zugesetzt. Der Zusatz von durch FIXa bzw. FXa spaltbaren Substraten ermöglicht deren Quantifizierung - und damit von FVIIa - mittels entsprechender Standardkurven. In einer anderen bevorzugten Ausführung können Aliquots des so generierten FIXa oder FXa in einem entsprechenden Gerinnungstest (Plasma, Mangelplasma) quantifiziert werden. Darüber hinaus können entsprechend andere FIXa- und FXa-Substrate chemischer oder biologischer Natur zur Quantifizierung und/oder Verstärkung der Kaskadenreaktion Verwendung finden.

In einer weiteren bevorzugten Verfahrensweise wird der an den wie oben beschrieben immobilisierten sTF gekoppelte FVII/FVIIa durch eine geeignete Lösung eluiert. Bevorzugt wird ein zweiwertige Ionen komplexierendes Agens, z.B. Citrat, Oxalat oder EDTA, zugesetzt. Das FVII/FVIIa enthaltende Eluat wird dann mit den oben beschriebenen Methoden getestet (ggfs. nach Zusatz von Kalzium, Lipiden): direkt (FVIIa-Substrat) oder indirekt (FX oder FIX und geeignete Substrate bzw. koagulometrisch). Die FVIIa- haltige Lösung kann - nun frei von störenden anderen (aktivierten) Faktoren - auch mittels eines Tests quantifiziert werden, der die Selektivität des sTF hinsichtlich der durch sTF/FVIIa initiierten FX-Aktivierung und der Folgereaktion (Gerinnung) ausnutzt. Ein solcher Test kann beispielsweise wie von Neuenschwander et al. beschrieben (J. Biol. Chem. 1992; 267: 14477-14482) ausgeführt werden.

Proben, die neben FVIIa auch andere aktivierte Faktoren enthalten (s.o.), können zu einer weiteren Generierung von FVIIa (ggfs. aus dem Überschuß des präsenten Faktors VII) und damit zu artifiziellen Ergebnissen führen. Zur Vorbeugung dieses Risikos kann der Probe vor Kontakt mit dem immobilisierten sTF Antithrombin III/Heparin zugesetzt werden. Da FVIIa durch ATIII/Hep. bei Raumtemperatur bzw. höheren Temperaturen im Vergleich zu potentiell störenden anderen Faktoren (FIIa, FIXa, FXa etc.) nur langsam inhibiert wird, können die letzteren blockiert werden, ohne den Gehalt an FVIIa wesentlich zu beeinflussen. TF-gebundener FVIIa kann u.U. durch ATIII/Hep. besser inhibierbar sein als die freien Moleküle (ATIII reagiert jedoch ohne funktionelles Heparin nur sehr langsam, analog löslichem FVIIa). Daher kann vor Kontakt mit sTF das zugesetzte Heparin mittels bekannter Reagenzien wie Protaminsulfat/Polybren® neutralisiert werden und anschließend wie oben beschrieben verfahren werden.
Für diesen Verfahrensschritt eignen sich ebenfalls reversible Inhibitoren , z.B. Benzamidin, und andere Kofaktor-abhängige Inhibitoren, die selbst oder deren Akzeleratoren neutralisiert werden können (beispielsweise Heparin-Kofaktor II / Heparin).

Bei Proben, die FVII und FVIIa enthalten, ist häufig nicht nur die absolute Konzentration von FVIIa von Interesse, sondern auch dessen Anteil am gesamten FVII/FVIIa-Gehalt. Für die Bestimmung des gesamten FVII-Gehaltes eignen sich Aktivitätstests oder entsprechende Antigennachweissysteme (z.B. FVII-ELISA). Im Zuge des o.g. Verfahrens ist es jedoch praktikabel, die Gesamtheit der an die Festphase (z.B. Mikrotiterplatten-Vertiefung, Röhrchen, Gelmatrix etc.) gebundenen (und danach ggfs. eluierten) Moleküle zu quantifizieren und in Verhältnis zur nachgewiesenen FVIIa-Konzentration zu setzen:
Die gebundene Gesamtmenge kann durch Aktivierung aller noch als Zymogen vorliegenden sTF-gebundenen FVII-Moleküle (bzw. sTF-FVII) und anschließende Zugabe eines o.g. FVIIa-Substrates (Messung wie beschrieben) bestimmt werden. Als Aktivator können z.B. Thrombin, FXa, FIXa, Präkallikreinaktivator, TF/Lipid (jeweils vorzugsweise in Gegenwart von Kalzium) etc. verwendet werden. Vor Zugabe des FVIIa-Substrates sollte dieser Aktivator durch gründliches Waschen der Festphase entfernt werden, um unspezifische/ungewollte Substratumsätze zu vermeiden. In Frage kommen ebenfalls Inhibitoren, die diese Aktivatoren inhibieren, jedoch den sTF-gebundnen FVIIa nicht blockieren. Als Beispiel soll Thrombin genannt werden, das spezifisch mittels r-Hirudin inhibierbar ist.
Im Falle der Elution von FVII/FVIIa empfiehlt sich die Bestimmung der Gesamtmenge mittels eines chromogenen Tests. Durch Zugabe von z.B. komplettem TF/Lipid, Kalzium, FX und einem durch FX spaltbaren Substrat, Inkubation und Messung des Substratumsatzes, kann die Summe aus FVII und FVIIa ermittelt werden (Seligsohn et al.; Blood 1978; 52: 978-988).

Die Erfindung wird durch die folgenden Beispiele erläutert.

### Beispiel 1: Bindung von FVII/FVIIa an mit Fc-sTF beladene (Protein A - beschichtete) Mikrotiterplatten und Bestimmung von FVIIa

### Material

Mit Protein A beschichtete Mikrotiterplatten (Sättigung freier Valenzen mit einer Albumin haltigen Lösung und anschließendes Waschen) wurden mit Fc-sTF durch Inkubation für 1 Stunde bei 37°C beladen, die Platten gewaschen und für den FVIIa-Test verwendet. Für die Erstellung einer Standardkurve wurde aus Plasma hoch gereinigter FVIIa bekannter Aktivität/Antigengehalt verwendet.

### Bindung von FVIIa

In die Vertiefungen der Mikrotiterplatten (wells) wurde je ein Teil (z.B. 50µl) eines CaCl₂-haltigen Puffers (30 mM) (Vorlagepuffer) vorgelegt und dazu je ein Teil der zu bestimmenden Probe pipettiert. Im Falle der Standardkurven-Erstellung enthielt die Probe steigende Konzentrationen FVIIa im Bereich von 0 bis 3000 ng/ml. Der Vorlagepuffer enthielt zudem Albumin und Detergenz, um unspezifische Bindungen an Gefäßwände zu verhindern (darüber hinaus kann diesem Puffer Protamin zugegeben werden, wenn die Probe Heparin enthält). Nach einstündiger Inkubation bei 37°C wurden die Platten mehrmals mit einer Kalzium-haltigen Pufferlösung gewaschen.

### Messung der FVIIa-Aktivitäten

In jede Vertiefung der Mikrotiterplatte wurde ein definiertes Volumen (100 µl) einer Lösung pipettiert, die Kalzium (5 mM CaCl₂), gerinnungsaktives Lipid/Lipidgemisch (beispielsweise Pathromtin®, Behringwerke AG), gereinigten FX (1IE/ml) und ein durch FXa spaltbares Substrat (4 mM S-2222, Fa. Chromogenix AB/Schweden) enthielt. Zur Verhinderung von unspezifischen Substratspaltungen oder Aktivierungsreaktionen durch Spuren Thrombin (die mit dem FX eingeschleppt werden könnten) wurden 10µg/ml r-Hirudin (HBW 023, Behringwerke AG/Hoechst AG) zugesetzt. Nach einstündiger Inkubation bei 37°C wurde die Reaktion durch Zugabe von 100µl/Vertiefung 10%iger Essigsäure gestoppt und die OD₄₀₅ photometrisch bestimmt. Kontrollansätze a) ohne Probe b) mit Lipid/Substrat/Hirudin, aber ohne FX und c) ohne Fc-sTF wurden mitgeführt.

In diesem Beispiel wurde die Standardkurve zwischen 0 und 3000 ng/ml FVIIa erstellt. Die OD405 nach einer Stunde sind in der folgenden Tabelle wiedergegben:

| **FVIIa (ng/ml)** | **OD405 (Mittelwerte von Doppelbestimmungen)** |
|---|---|
| 0 | 0 |
| 30 | 0,08 |
| 100 | 0,20 |
| 300 | 0,40 |
| 1000 | 0,73 |
| 3000 | 0,93 |
| Kontrollen a/b/c | 0 - 0,015 |

### Beispiel 2: Bindung an Fc-sTF, gekoppelt an Protein A-Sepharose, und Elution: Bestimmung von FVIIa und der Gesamtkonzentrationen von FVII/FVIIa

### Bindung und Elution von FVII/FVIIa

Protein A-Sepharose wurde mit Fc-sTF beladen, die Matrix gewaschen und für die Bindung von FVII/FVIIa verwendet.

Jeweils 0,5 ml der mit Fc-sTF beladenen (Protein A-beschichtete) Sepharose wurden in kleine Säulen gefüllt und mit CaCl₂(5 mM)-haltigem Puffer äquilibriert. Die FVII/FVIIa-haltigen Lösungen wurden mit CaCl₂-haltigem Puffer verdünnt. Jeweils 0,5 ml dieser Lösungen wurde bei Raumtemperatur auf die Säule aufgetragen. Die Säulchen wurden mit einem CaCl₂-haltigen Puffer gewaschen. Die Elution erfolgte mit einer Natrium-Citrat (50 mM) haltigen Pufferlösung.

### Quantifizierung von FVIIa und der Gesamt-Konzentration FVII/FVIIa

FVIIa, eluiert von der o.g. Matrix, wurde mit einem Testsystem nach Neuenschwander et al. (J. Biol. Chem. 1992; 267: 14477-14482) quantifiziert: verschiedene Probenverdünnungen wurden mit einem FVII-Mangelplasma gemischt. In Gegenwart von sTF, Lipid und Kalzium wurde die Zeit bis zur Gerinnung mit einem Koagulometer nach Schnitger und Gross ermittelt und die FVIIa-Gehalte anhand einer Standardkurve bestimmt. Zur Erstellung einer Standardkurve wurden steigende Konzentrationen rFVIIa von NIBSC (UK) verwendet.

Die Bestimmung der Gesamt-Gehalte an FVII/FVIIa wurde modifiziert nach Seligsohn et al. (s.o.) durchgeführt: verschiedene Verdünnungen der Proben wurden mit komplettem TF/Lipidgemisch, Kalzium und FX inkubiert. Die Generierung von FXa wurde nach 3 min Inkubation durch Zugabe von EDTA gestoppt und die ΔOD/min (chromogenes Substrat S-2765; Chromogenix AB) bestimmt. Als Referenzen dienten steigende Konzentrationen von FVII bzw. FVIIa, anhand derer die Probengehalte ermittelt wurden.

### Ansätze

F VII wurde auf 0,5 E/ml (nach Gesamttest) mit Kalzium-haltiger Pufferlösung eingestellt (siehe Tab.2: A-Mat=Ausgangsmaterial). Weitere Proben steigender FVIIa-Gehalte wurden hergestellt, wobei die Konzentrationen FVII+FVIIa auf 0,5 E/ml (nach Gesamttest) eingestellt wurden.

### Ergebnis

Wie Tab. 2 zeigt, wurden jeweils etwa 90% der applizierten FVII/VIIa-Gesamtaktivitäten und FVIIa-Aktivitäten (bezogen auf die Ausgangsmengen) in den Eluaten der sTF-Säulen wiedergefunden. Da das Ziel der Untersuchungen die Bestimmung des FVIIa-Anteiles an der Gesamtkonzentration einer FVII- und FVIIa-haltigen Probe war (ausgedrückt als Verhältnis FVIIa:Gesamt-FVII/VIIa), spielen die absolut wiedergefundenen Mengen der Faktoren keine Rolle, solange die Detektierbarkeit gewährleistet bleibt. Wie die in (Spalte 3) der Tabelle aufgeführten Faktoren FVIIa:Gesamt-FVII/VIIa verdeutlichen, findet durch diese Affinitätschromatografie keine Aktivierung des FVII-Anteiles (durch FVIIa-Triggerung) statt- eine wichtige Vorraussetzung für das Funktionieren dieses Testsystemes.

Bereits der vermeintlich reine FVII enthielt einen detektierbaren Anteil an FVIIa, wie Tab.2 (*) zeigt.
Durch sukzessive Reduktion des FVII-Anteiles und Ersatz durch FVIIa blieb die Summe FVII+VIIa im Gesamttest konstant, stieg jedoch erwartungsgemäß im FVIIa-Test. Entsprechend vergrößerte sich der Faktor FVIIa:Gesamt-FVII+VIIa mit steigendem FVIIa-Anteil. Der Vergleich dieser Faktoren, nämlich A-Mat zu Eluat war (im Rahmen der Testschwankungen) jeweils identisch für die verschiedenen Gemische und zeigte die Verläßlichkeit des Testsystemes.

**Tabelle 2**

| FVIIa-Anteil FVII/VIIa (%) | Gesamttest (E FVII+VIIa/ml) | | FVIIa-Test (E FVIIa/ml) | | FVIIa:Ges.-(FVIIa-E/Gesamt-E) | |
|---|---|---|---|---|---|---|
| | A-Mat | Eluat | A-Mat | Eluat | A-Mat | Eluat |
| 0 | 0,50 | 0,39 | 0,07* | 0,050 | 0,14 | 0,13 |
| 2 | 0,55 | 0,39 | 0,30 | 0,225 | 0,55 | 0,58 |
| 5 | 0,50 | 0,38 | 0,62 | 0,488 | 1,24 | 1,28 |
| 20 | 0,55 | 0,42 | 2,38 | 1,820 | 4,33 | 4,33 |
| 100 | 0,50 | 0,35 | 10,15 | 6,500 | 20,30 | 18,57 |

## Patentansprüche

1. Verfahren zum Nachweis von aktiviertem Faktor VII (FVIIa) in Proteinlösungen umfassend die Schritte
a) Bindung des FVIIa an immobilisiertes lösliches Thromboplastin (sTF) und
b) Nachweis des gebundenen FVIIa durch direkte oder indirekte Bestimmung oder Nachweis des eluierten FVIIa durch direkte oder indirekte Bestimmung.

2. Verfahren nach Anspruch 1, dadurch gekennzeichnet, daß vor der Bindung an immobilisiertes lösliches Thromboplastin das Ausgangsmaterial (die Auftragslösung) mit Antithrombin III/Heparin inkubiert und anschließend das Heparin neutralisiert wird.

3. Verfahren nach Anspruch 1 oder 2, dadurch gekennzeichnet, daß das immobilisierte lösliche Thromboplastin aus gentechnisch hergestelltem sTF-Fc und Bindung an eine Fc-bindende Säule hergestellt wird.

4. Verfahren nach Anspruch 3, dadurch gekennzeichnet, daß eine Protein-A-Säule zur sTF-Fc-Bindung eingesetzt wird.

5. Verfahren nach Anspruch 1, 2, 3 oder 4, dadurch gekennzeichnet, daß die FVIIa-Aktivität amidolytisch bestimmt wird.

6. Verfahren nach einem der vorhergehenden Ansprüche, dadurch gekennzeichnet, daß die FVIIa-Menge über die Generierung von FXa und/oder FIXa bestimmt wird.

7. Verfahren nach Anspruch 1b), dadurch gekennzeichnet, daß die FVIIa-Menge mit Hilfe eines gelösten löslichen Thromboplastins koagulometrisch quantifiziert wird.

8. Verfahren nach Anspruch 1b), dadurch gekennzeichnet, daß die FVIIa-Menge durch Ermittlung des Verhältnisses Clotting-Aktivität/Gesamt-Aktivität (chromogen) bestimmt wird.
